# EUROPEAN PATENT SPECIFICATION

(11) **EP 2 461 907 B1**
(45) Date of publication and mention of the grant of the patent: **14.09.2016**
(21) Application number: 10745056.1
(22) Date of filing: 05.08.2010
(51) Int. Cl.: C01B 39/42, B01J 29/70, C07C 6/12, C10G 45/64

(54) **SYNTHESIS AND USE OF ZSM-12**
SYNTHESE UND VERWENDUNG VON ZSM-12
SYNTHÈSE ET UTILISATION DE LA ZSM-12

(30) Priority: 07.08.2009 US 273711 P; 04.08.2010 US 850060
(43) Date of publication of application: 13.06.2012
(73) Proprietor: ExxonMobil Research and Engineering Company, Annandale, NJ 08801-0900 (US)
(72) Inventor: LAI, Frank, W., Bridgewater NJ 08807 (US); ELKS, Jeffrey, T., Geneva IL 60134 (US); KAY, Robert, E., Easton PA 18045 (US)
(74) Representative: Mareschal, Anne
(86) International application number: PCT/US2010/044527
(87) International publication number: WO 2011/017506

(56) References cited:
- EP-A2- 1 110 911
- US-A1- 2003 091 504
- US-A1- 2008 035 525
- US-B2- 6 893 624
- GOPAL S ET AL: "Synthesis of Al-rich ZSM-12 using TEAOH as template", MICROPOROUS AND MESOPOROUS MATERIALS, ELSEVIER SCIENCE PUBLISHING, NEW YORK, US, vol. 49, no. 1-3, 15 November 2001 (2001-11-15), pages 149-156, XP004341582, ISSN: 1387-1811, DOI: DOI:10.1016/S1387-1811(01)00412-7
- YOO K ET AL: "TEABr directed synthesis of ZSM-12 and its NMR characterization", MICROPOROUS AND MESOPOROUS MATERIALS, ELSEVIER SCIENCE PUBLISHING, NEW YORK, US, vol. 60, no. 1-3, 19 June 2003 (2003-06-19), pages 57-68, XP004430813, ISSN: 1387-1811, DOI: DOI:10.1016/S1387-1811(03)00317-2
- KATOVIC A ET AL: "PRELIMINARY STUDY OF ZSM-12 ZEOLITE SYNTHESIS", CHEMISTRY EXPRESS, KINKI CHEMICAL SOCIETY, JP, vol. 61, no. 12, 1 January 1991 (1991-01-01), pages 969-972, XP009033372, ISSN: 0911-9566
- TOKTAREV A V ET AL: "STUDIES ON CRYSTALLIZATION OF ZSM-12 TYPE ZEOLITE", STUDIES IN SURFACE SCIENCE AND CATALYSIS, ELSEVIER BV, NL, vol. 105, 1 January 1997 (1997-01-01), page 333, XP009033424, ISSN: 0167-2991, DOI: DOI:10.1016/S0167-2991(97)80573-3

## Description

### FIELD

This invention relates to a process for the synthesis of ZSM-12 with controlled mesoporosity and to the use of the resultant ZSM-12 in catalytic processes, particularly in the conversion of C₉+ aromatic hydrocarbons to xylenes.

### BACKGROUND

ZSM-12 and its conventional preparation in the presence of a tetramethylammonium or tetraethylammonium directing agent are taught by U.S. Patent No. 3,832,449. ZSM-12 has a distinctive X-ray diffraction pattern which distinguishes it from other known crystalline materials.

There is a growing demand for ZSM-12-type catalyst materials in the chemical and refining fields. As a result there is significant interest in developing improved techniques for the synthesis of ZSM-12.

U.S. Patent No. 4,391,785 discloses a method for the synthesis of ZSM-12 from a reaction mixture comprising, as a directing agent, a compound selected from the group consisting of a dimethylpyridinium halide and a dimethylpyrrolidinium halide.

U.S. Patent Nos. 4,452,769 and 4,537,758 disclose methods for synthesizing ZSM-12 from a reaction mixture containing methyltriethylammonium ions as the directing agent. These patents are primarily directed to producing high SiO₂/Al₂O₃ ratio forms of ZSM-12, greater than 80 in the case of the '769 patent and greater than 200 in the case of the '758 patent. Moreover, one of the stated advantages in the '769 patent of using methyltriethylammonium ions as the directing agent is the production of large crystal size materials.

Other organic directing agents that have been used to synthesize ZSM-12 include DABCO-Cₙ-diquat ions where n = 4, 5, 6 or 10 (see U.S. Patent No. 4,482,531), bis(dimethylpiperidinium)trimethylene ions (see U.S. Patent No. 4,539,193), benzyltriethylammonium ions (see U.S. Patent No. 4,552,738), dibenzyldiethylammonium ions (see EP-A-167,232), dimethyldiethylammonium ions (see U.S. Patent No. 4,552,739), benzyltrimethylammonium ions (see U.S. Patent No. 4,585,637), bis(N-methylpyridyl) ethylinium ions (see U.S. Patent No. 4,585,746), hexamethyleneimine (U.S. Patent No. 5,021,141), and decamethonium ions (see U.S. Patent No. 5,192,521) bis(methylpyrrolidinium) diquat-n ions where where n = 4, 5, or 6.

Although influenced by variables such as the silica/alumina molar ratio of the reaction mixture, temperature and stirring, the crystal morphology of synthetic zeolites, such as ZSM-12, is mainly dominated by the choice of directing agent used in the crystallization. For example, in the case of ZSM-12, needle-shaped crystals can be produced using a benzyltrimethylammonium directing agent, rice-shaped crystals can be made in the presence of tetraethylammonium salts, and bundles of hexagonal platelets can be prepared from with a hexamethyleneimine directing agent. The control of zeolite crystal morphology is very important from the standpoint of activity and stability enhancement. For most catalytic applications, small crystal size is desirable for high activity and stability because of the higher surface area, and hence the shorter diffusion path, of small crystal materials.

For example, U.S. Patent No. 6,893,624 discloses the synthesis of ZSM-12 having a silica to alumina molar ratio less than 60, an average crystal size of the material is less than 0.1 micron and a Diffusion Parameter for mesitylene of at least 1000x10⁻⁶ sec⁻¹ when measured at a temperature of 100°C and a mesitylene pressure of 2 torr. The synthesis is conducted by crystallizing a reaction mixture comprising a source of an oxide of a trivalent element X , a source of an oxide of a tetravalent element Y, methyltriethylammonium cations (R) as a template, an alkali metal and/or alkaline earth metal ion source M having the valency n and water; wherein mixture has the following composition in terms of the molar ratios: YO₂/X₂O₃ is 40 to 80, H₂O/YO₂ is 15 to 40, OH⁻/ YO₂ is 0.15 to 0.4, M/YO₂ is 0.15 to 0.4 and R/ YO₂ is 0.15 to 0.4. The crystallization is carried out at a temperature of 170°C or less for a time of about 50 to 500 hr.

In addition, US Patent Application Publication No. 2008/0035525 discloses a process for producing ZSM-12 having a primary crystal size of <0.1 µm; and a specific volume, determined by mercury porosimetry at a maximum pressure of 4000 bar, of 30-200 MM³/g in a pore radius range of 4-10 nm. The process involves crystallization of a synthesis gel composition comprising an aluminum source, precipitated silica as a silicon source, TEA⁺ as a template, an alkali metal and/or alkaline earth metal ion source M having the valency n and water; in which the molar H₂O:SiO₂ ratio is selected between 5 and 15, the molar M_{2/n}O:SiO₂ ratio is within the range from 0.01 to 0.045, molar TEA⁺/SiO₂ ratio is between about 0.10 and 0.18, the molar SiO₂/Al₂O₃ ratio is within a range from 50 to 150. The crystallization is carried out at a temperature of from about 120 to 200°C, preferably from about 140 to 180°C, for a time of about 50 to 500 hr, in particular from about 100 to 250 hr.

According to the present invention it has now been found that, by modifying the reaction mixture and reaction temperature, the time required for synthesis of ZSM-12 can be reduced to less than 50 hours thereby increasing production throughput as well as reducing the overall cost of synthesis. In addition, depending on the synthesis conditions, the resultant ZSM-12 is in the form of agglomerates of closely or loosely packed, small ZSM-12 crystals.

It is to be appreciated that, although ZSM-12 is normally synthesized as an aluminosilicate, the framework aluminum can be partially or completely replaced by other trivalent elements, such as boron, iron and/or gallium, and the framework silicon can be partially or completely replaced by other tetravalent elements such as germanium.

### SUMMARY

In one aspect, the invention resides in a process for synthesizing a porous, crystalline material having the framework structure of ZSM-12 of the formula:

(n)YO₂: X₂O₃

wherein X is a trivalent element, Y is a tetravalent element and n is between 80 and 300, the process comprising:
(a) preparing a mixture capable of forming said material, said mixture comprising sources of alkali or alkaline earth metal (M), an oxide of trivalent element (X), an oxide of tetravalent element (Y), hydroxyl ions (OH⁻), water and tetraethylammonium cations (R), wherein said mixture has a composition, in terms of mole ratios, within the following ranges:
   YO₂/X₂O₃ = 100 to 300;
   H₂O/YO₂ = 5 to 15;
   OH⁻/YO₂ = 0.10 to 0.30;
   M/YO₂ = 0.1 to 0.2; and
   R/YO₂ = 0.10 to 0.20;
(b) reacting said mixture at a temperature of at least 300° F (149° C) to form crystals of said material; and
(c) terminating (b) after a period of time of less than 50 hours and recovering said crystalline material.

In one embodiment, said temperature is 300° F (149° C) to 330°F (166°C), such as 320° F (160° C).

Conveniently, (b) is terminated after a period of time of less than or equal to 40 hours, such as from 30 hours to 40 hours.

Conveniently, the mole ratio of H₂O/YO₂ is between 10 and 14.

Conveniently, the mole ratio of OH⁻/YO₂ is 0.20 to 0.30.

Conveniently, the mole ratio of M/YO₂ is less than or equal to 0.15, such as less than 0.13.

Conveniently, the mole ratio of R/YO₂ is less than or equal to 0.15. Conveniently, X comprises aluminium and Y comprises silicon.

Generally, said crystalline material comprises agglomerates of ZSM-12 crystals having a median mesopore radius between 10Å and 40Å, typically between 10 Å and 35 Å, as measured by argon-physisorption. In addition, the ZSM-12 crystals typically have an external surface area between 10 and 20% of their total surface area.

In a further aspect, the invention resides in use of the ZSM-12 produced herein in the conversion of C₉+ aromatic hydrocarbons to xylenes, comprising contacting a feed comprising at least one C₉⁺ aromatic hydrocarbon under conversion conditions with a catalyst comprising the porous, crystalline material of the above mentioned aspect of the invention.

### DESCRIPTION OF THE DRAWINGS

Figures 1 (a) and (b) provide an argon-physiosorption spectrum and a scanning electron micrograph (SEM), respectively, of the product of Example 1.
Figures 2 (a) and (b) provide an argori-physiosorption spectrum and a scanning electron micrograph (SEM), respectively, of the product of Example 3.
Figures 3 (a) and (b) provide an argon-physiosorption spectrum and a scanning electron micrograph (SEM), respectively, of the product of Example 4.
Figures 4 (a) and (b) provide an argon-physiosorption spectrum and a scanning electron micrograph (SEM), respectively, of the product of Example 5.

### DETAILED DESCRIPTION OF THE EMBODIMENTS

Described herein is a high solids, high temperature process for the synthesis of ZSM-12 which allows for the high throughput and short cycle time synthesis of agglomerates of loosely packed small ZSM-12 crystals, typically having an average crystal size of less 0.05 micron. Also described herein is use of the resultant ZSM-12 crystals in the conversion of C₉+ aromatic hydrocarbons to xylenes.

In the present process, a synthesis mixture is prepared containing sources of alkali or alkaline earth metal (M) cations, normally sodium, an oxide of a trivalent element (X), normally alumina, an oxide of a tetravalent element (Y), normally silica, tetraethylammonium cations (R), hydroxyl ions and water. The synthesis mixture has a composition, expressed in terms of mole ratios of oxides, as follows:

**Table 1**

| Component | Useful | Preferred |
|---|---|---|
| YO₂/X₂O₃ | 100 to 300 | 150 to 250 |
| H₂O/YO₂ | 5 to 15 | 10 to 14 |
| OH⁻/YO₂ | 0.1 to 0.3 | to 0.25 |
| M/YO₂ | 0.1 to 0.2 | 0.1 to 0.2 |
| R/YO₂ | 0.1 to 0.2 | 0.1 to 0.15 |

In one embodiment, the mole ratio of M/YO₂ is less than 0.13.

Some or all of the alkali or alkaline earth metal, M, can be added as a metal salt, such as NaCl, and NaBr, rather than as the hydroxide, to assist in maintaining a low OH⁻/YO₂ ratio to improve silica utilization (yield) while maintaining the M/YO₂ ratio within the desired range.

The synthesis method of the invention functions with or without added nucleating seeds. In a preferred embodiment, the reaction mixture contains 0.05 to 5 wt% nucleating seeds of ZSM-12.

Crystallization is carried out under either stirred or static conditions, preferably stirred conditions, at a relatively high temperature of at least 300° F (149° C), and preferably 300° F (149° C) to 330°F (166°C), such as ww 320° F (160° C). The crystallization is conducted for a period of time of less than 50 hours, generally less than or equal to 40 hours, such as from 30 hours to 40 hours, whereafter the resultant ZSM-12 crystals are separated from the mother liquor and recovered. By virtue of its high solids content and short crystallization time, the present process allows for the high throughput synthesis of ZSM-12.

The ZSM-12 produced by the present process is in the form of agglomerates of loosely packed small crystals, typically having an average crystal size of less 0.05 micron. In particular, agglomerates of closely packed small crystals tend to be produced at Na/Si molar ratios of less than or equal to 0.1 and synthesis temperatures at least 320° F (160° C). In contrast, agglomerates of loosely packed small crystals tend to be produced at Na/Si molar ratios of greater than to 0.1 and higher synthesis temperatures of less than 320° F (160° C).

Close packing of the ZSM-12 crystals is demonstrated by the median radius of the intercrystalline mesopores being in the range of 10Å and 30Å, typically between 15 Å and 25 Å, as measured by argon-physisorption. Loose packing of the ZSM-12 crystals is demonstrated by the median radius of the intercrystalline mesopores being in the range of 20Å and 50Å, typically between 25 Å and 45 Å, as measured by argon-physisorption. In addition, the closely packed ZSM-12 crystals typically have an external surface area between 10 and 20% of their total surface area, while the loosely packed crystals have an external surface area greater than 20% of their total surface area.

In its as-synthesized form, the ZSM-12 produced hereby contains alkali or alkaline earth metal (M) cations as well as the organic material used to direct its synthesis. Prior to use of the ZSM-12 as a catalyst or adsorbent, the as-synthesized material is normally treated to remove part or all of the organic constituent. This is conveniently effected by heating the as-synthesized material at a temperature of from 250°C to 550°C for from 1 hour to 48 hours.

To the extent desired, the original sodium and/or potassium cations of the as-synthesized material can be replaced in accordance with techniques well known in the art, at least in part, by ion exchange with other cations. Preferred replacing cations include metal ions, hydrogen ions, hydrogen precursor, e.g., ammonium ions and mixtures thereof. Particularly preferred cations are those which tailor the catalytic activity for certain hydrocarbon conversion reactions. These include hydrogen, rare earth metals and metals of Groups IIA, IIIA, IVA, VA, IB, IIB, IIIB, IVB, VB, VIB, VIIB and VIII of the Periodic Table of the Elements.

The ZSM-12 produced according to the present process has an X-ray diffraction pattern characterized by the X-ray diffraction lines in Table 1 below:

**Table 1**

| D-spacing (Å) | Relative Intensity [100 x I/Io] |
|---|---|
| 11.9 ± 0.2 | m |
| 10.1 ± 0.2 | m |
| 4.76 ± 0.1 | w |
| 4.29 ± 0.08 | vs |
| 3.98 ± 0.08 | m |
| 3.87 ± 0.07 | vs |
| 3.49 ± 0.07 | w |
| 3.38 ± 0.07 | m |
| 3.20 ± 0.06 | w |
| 3.05 ± 0.05 | w |
| 2.54 ± 0.03 | w |

These X-ray diffraction data were collected with a Scintag diffractometer using copper K-alpha radiation. The diffraction data were recorded by step-scanning at 0.02 degrees of two-theta, where theta is the Bragg angle, and a counting time of 1 second for each step. The interplanar spacings, d's, were calculated in Angstrom units (A), and the relative intensities of the lines, I/ Io, where Io is one-hundredth of the intensity of the strongest line, above background, were derived with the use of a profile fitting routine (or second derivative algorithm). The intensities are uncorrected for Lorentz and polarization effects. The relative intensities are given in terms of the symbols vs=very strong (75-100), s=strong (50-74), m=medium (25-49) and w=weak (0-24). It should be understood that diffraction data listed for this sample as single lines may consist of multiple overlapping lines which under certain conditions, such as differences in crystallite sizes or very high experimental resolution or crystallographic change, may appear as resolved or partially resolved lines. Typically, crystallographic changes can include minor changes in unit cell parameters and/or a change in crystal symmetry, without a change in topology of the structure. These minor effects, including changes in relative intensities, can also occur as a result of differences in cation content, framework composition, nature and degree of pore filling, and thermal and/or hydrothermal history.

The crystalline material ZSM-12 produced according to the present process has a composition involving the molar relationship:

X₂O₃ : (n)YO₂

wherein X is a trivalent element, such as aluminum, boron, iron, indium and/or gallium, preferably aluminum; Y is a tetravalent element, such as silicon, tin and/or germanium, preferably silicon; and n is between 80 and 250. In its as-synthesized form, the crystalline material of the invention has a formula, on an anhydrous basis and in terms of moles of oxides per n moles of YO₂, as follows:
(0.01 to 2)M₂O:(0.01 to 2)R₂O: X₂O₃ :(n)YO₂
wherein M is an alkaline or alkaline earth metal, and is preferably sodium, and R is the tetraethylammonium cation. The M and R components are associated with the material as a result of their presence during crystallization, and are easily removed by post-crystallization methods as described above.

The crystalline ZSM-12 described herein, when employed either as an adsorbent or as a catalyst in an organic compound conversion process should be dehydrated, at least partially. This can be done by heating to a temperature in the range of 200°C to 370°C in an atmosphere such as air or nitrogen, and at atmospheric, subatmospheric or superatmospheric pressures for between 30 minutes and 48 hours. Dehydration can also be performed at room temperature merely by placing the ZSM-12 in a vacuum, but a longer time is required to obtain a sufficient amount of dehydration.

Synthetic ZSM-12 crystals prepared in accordance herewith can be used either in the as-synthesized form, the hydrogen form or another univalent or multivalent cationic form. It can also be used in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such components can be exchanged into the composition, impregnated therein or physically intimately admixed therewith. Such components can be impregnated in or on to the ZSM-12 such as, for example, by, in the case of platinum, treating the material with a platinum metal-containing ion. Suitable platinum compounds for this purpose include chloroplatinic acid, platinous chloride and various compounds containing the platinum amine complex. Combinations of metals and methods for their introduction can also be used.

When used as a catalyst, it may be desirable to incorporate the present ZSM-12 with another material resistant to the temperatures and other conditions employed in certain organic conversion processes. Such matrix materials include active and inactive materials and synthetic or naturally occurring zeolites as well as inorganic materials such as clays, silica and/or metal oxides, e.g. alumina, titania and/or zirconia. The latter may be either naturally occurring or in the form of gelatinous precipitates, sols or gels including mixtures of silica and metal oxides. Use of a material in conjunction with the ZSM-12, i.e. combined therewith, which is active, may enhance the conversion and/or selectivity of the catalyst in certain organic conversion processes. Inactive materials suitably serve as diluents to control the amount of conversion in a given process so that products can be obtained economically and orderly without employing other means for controlling the rate or reaction. Frequently, crystalline catalytic materials have been incorporated into naturally occurring clays, e.g. bentonite and kaolin. These materials, i.e. clays, oxides, etc., function, in part, as binders for the catalyst. It is desirable to provide a catalyst having good crush strength because in a petroleum refinery the catalyst is often subjected to rough handling, which tends to break the catalyst down into powder-like materials which cause problems in processing.

Naturally occurring clays which can be composited with the hereby synthesized crystalline material include the montmorillonite and kaolin families which include the subbentonites, and the kaolins commonly known as Dixie, McNamee, Georgia and Florida clays, or others in which the main mineral constituent is halloysite, kaolinite, dickite, nacrite or anauxite. Such clays can be used in the raw state as originally mined or initially subjected to calcination, acid treatment or chemical modification.

In addition to the foregoing materials, the present crystals can be composited with a porous matrix material such as silica-alumina, silica-titania, alumina-titania, silica-magnesia, silica-zirconia, silica-thoria, silica-beryllia, as well as ternary compositions such as silica-alumina-thoria, silica-alumina-zirconia, silica-alumina-magnesia and silica-magnesia-zirconia. The matrix can be in the form of a cogel. A mixture of these components could also be used.

The relative proportions of ZSM-12 and matrix vary widely with the ZSM-12 content ranging from 1 to 90 percent by weight, and more usually in the range of 2 to 50 percent by weight of the composite.

Aluminosilicate ZSM-12 produced by the present process is useful as a catalyst in organic compound, and in particular hydrocarbon, conversion reactions where high activity is important. In particular, when combined with a hydrogenation component, such as platinum, palladium or rhenium, the ZSM-12 is useful in the catalytic conversion of C₉+ alkylaromatic hydrocarbons, either alone or in the presence of toluene and/or benzene, to produce xylenes. Such conversion is typically effected at a temperature of from 650 to about 950°F (340 to 510°C), and preferably from 750 to 850°F (400 to 450°C), a pressure of from 100 to 600 psig (790 to 4240kPa), and preferably from 200 to 500 psig (1480 to 3550kPa), a weight hourly space velocity (WHSV) of between 0.1 and 200 hr⁻¹, and preferably between 0.5 and 20 hr⁻¹, and a hydrogen, H₂, to hydrocarbon, HC, molar ratio of between 1 and 5, and preferably from 1 to 3.

Where the ZSM-12 described herein is used in the catalytic conversion of C₉+ alkylaromatic hydrocarbons, the ZSM-12 may be used in combination with a second molecular sieve having a constraint index of 3 to 12, such as ZSM-5, ZSM-11, ZSM-22, ZSM-23, ZSM-35, ZSM-48, ZSM-57 and ZSM-58. The ZSM-12 and second molecular sieve may be arranged in separate catalyst beds, with the feed cascading from the catalyst bed containing the ZSM-12 to the bed containing the second molecular sieve. Alternatively, the ZSM-12 and second molecular sieve can be combined in a single catalyst bed.

In order to more fully illustrate the nature of the invention and the manner of practicing same, the following Examples are presented.

In the Examples and throughout this specification, porosity values were determined by argon physisorption using an Omnisorp Dynamic Physisorption Characterization system. Approximately 200-250 mgs of "as received" sample were charged to a clean, tared sample cell, dried for 16 hrs under 1x10⁻⁵ Torr vacuum and reweighed to determine the dry mass used for analysis. Analyses were made dynamically at an argon flowrate of 0.20 cc/min at the boiling point of liquid argon (-87°K). Full isotherms were generated by flowing argon to within 99.5% of the 1-atmosphere saturation pressure of argon and then removing the sorbed argon with vacuum to about 280 torr (37 kPa) of pressure. A classic BET model was used to calculate total surface area. A t-plot model using argon film thickness was used to calculate the micropore volume and external surface area of the samples. A BJH desorption model was used to generate the mesopore size distributions and approximate average mesopore size.

### Example 1 (Comparative)

A mixture having the following molar composition was prepared from water, TEAOH (35% aqueous solution), NaOH (50% aqueous solution), sodium aluminate (45% aqueous solution), and Ultrasil silica:

| | |
|---|---|
| SiO₂/Al₂O₃ | ~250 |
| H₂O/SiO₂ | = 14 |
| OH⁻/SiO₂ | = 0.205 |
| Na⁺/SiO₂ | = 0.075 |
| TEA/SiO₂ | = 0.13 |

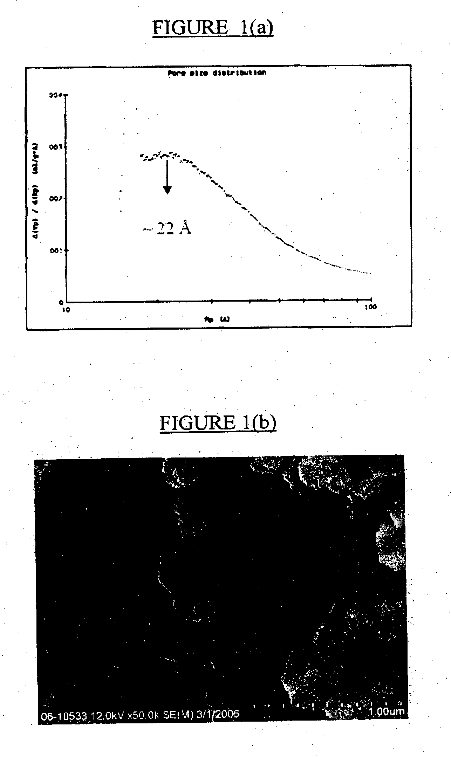

The mixture was reacted at 320°F (160°C) in a 5-gallon autoclave with stirring at 250 RPM for 36 hours. The product was filtered, washed with deionized (DI) water and dried at 250°F (120°C). The XRD pattern of the as-synthesized material showed the typical pure phase of ZSM-12 topology. The SEM, Figure 1 (b), of the as-synthesized material shows that the material was composed of more closely packed agglomerates of small crystals with an average crystal size of < 0.05 microns. Argon-physisorption shows that the H-form of the resulting product has an external surface area of about 17.5 % of the total surface area and a mesopore radius of about 22 Å, see Figure 1 (a).

### Example 2

A mixture having the following molar composition was prepared from water, TEAOH (35% aqueous solution), NaOH (50% aqueous solution), sodium aluminate (45% aqueous solution), and Ultrasil silica:

| | |
|---|---|
| SiO₂/Al₂O₃ | ~250 |
| H₂O/SiO₂ | = 14 |
| OH⁻/SiO₂ | ~ 0.24 |
| Na⁺/SiO₂ | = 0.125 |
| TEA/SiO₂ | = 0.13 |

The mixture was reacted at 320°F (160°C) in a 5-gallon autoclave with stirring at 250 RPM for 36 hours. T he product was filtered, washed with deionized (DI) water and dried at 250°F (120°C). The XRD pattern of the as-synthesized material showed the typical pure phase of ZSM-12 topology. The SEM of the as-synthesized material shows that the material was composed of less densely packed agglomerates of small crystals with an average crystal size of < 0.05 microns. Argon-physisorption demonstrates that the H-form of the resulting product shows an increased external surface area of about 19.5 % (compared to the total surface area) and larger (medium) mesopore radius of about 28 Å, as compared to the product of Example 1.

### Example 3

A mixture having the following molar composition was prepared from water, TEAOH (35% aqueous solution), NaOH (50% aqueous solution), sodium aluminate (45% aqueous solution), and Ultrasil silica:

| | |
|---|---|
| SiO₂/Al₂O₃ | ~250 |
| H₂O/SiO₂ | ~ 13.8 |
| OH⁻/SiO₂ | ~ 0.20 |
| Na⁺/SiO₂ | = 0.147 |
| TEA/SiO₂ | = 0.13 |

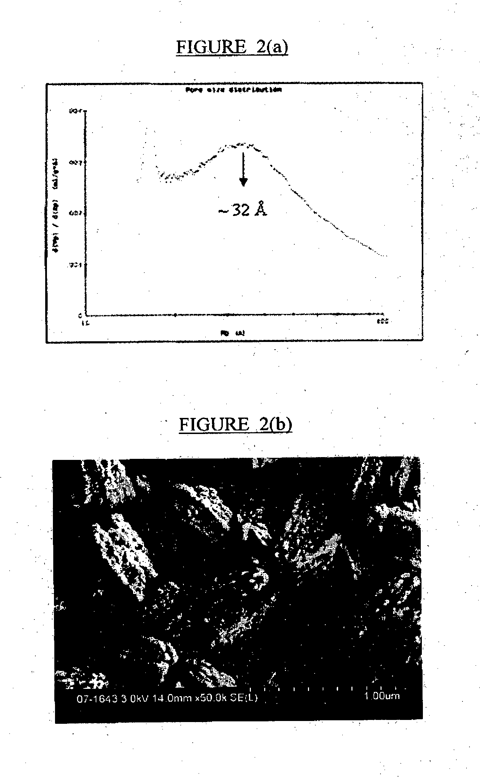

The mixture was reacted at 320°F (160°C) in a 5-gallon autoclave with stirring at 250 RPM for 36 hours. The product was filtered, washed with deionized (DI) water and dried at 250°F (120°C). The XRD pattern of the as-synthesized material showed the typical pure phase of ZSM-12 topology. The SEM, Figure 2 (b), of the as-synthesized material shows that the material was composed of less densely packed agglomerates of small crystals with an average crystal size of < 0.05 microns. Argon-physisorption demonstrates that the H-form of the resulting product shows an external surface area of about 20 % (as compared to total surface area) and an increased medium mesopore radius of about 32 Å (Figure 2 (a)) as compared to the product of Example 2.

### Example 4

A mixture having the following molar composition was prepared from water, TEAOH (35% aqueous solution), NaOH (50% aqueous solution), sodium aluminate (45% aqueous solution), and Ultrasil silica:

| | |
|---|---|
| SiO₂/Al₂O₃ | ~250 |
| H₂O/SiO₂ | ~ 13.8 |
| OH⁻/SiO₂ | ~ 0.20 |
| Na⁺/SiO₂ | ~ 0.15 |
| TEA/SiO₂ | = 0.13 |

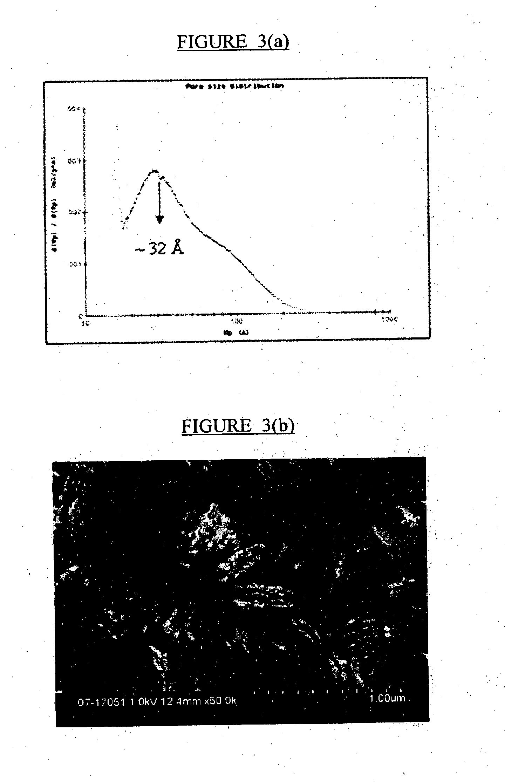

The mixture was reacted at 320°F (160°C) in a 5-gallon autoclave with stirring at 250 RPM for 36 hours. The product was filtered, washed with deionized (DI) water and dried at 250°F (120°C). The XRD pattern of the as-synthesized material showed the typical pure phase of ZSM-12 topology. T he SEM, Figure 3 (b), of the as-synthesized material shows that the material was composed of agglomerates of less densely packed small crystals with an average crystal size of < 0.05 microns. Argon-physisorption demonstrates that the H-form of the resulting product shows an of external surface area of about 20 % (as compared to total surface area) and an increased medium mesopore radius of about 32 Å (Figure 3 (a)) as compared to the product of Example 2.

### Example 5

A mixture having the following molar composition was prepared from water, TEAOH (35% aqueous solution), NaOH (50% aqueous solution), sodium aluminate (45% aqueous solution), and Ultrasil silica:

| | |
|---|---|
| SiO₂/Al₂O₃ | ~250 |
| H₂O/SiO₂ | ~ 13.8 |
| OH⁻/SiO₂ | ~ 0.20 |
| Na⁺/SiO₂ | ~ 0.15 |
| TEA/SiO₂ | = 0.13 |

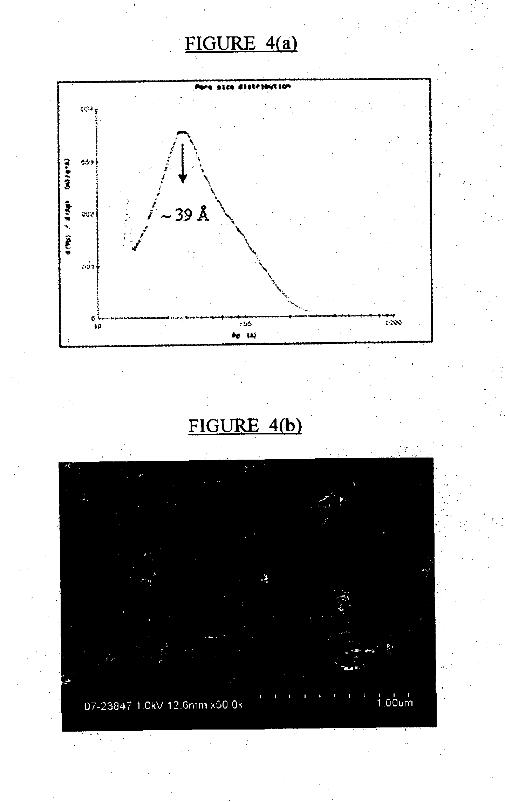

The mixture was reacted at 300°F (150°C) in a 5-gallon autoclave with stirring at 250 RPM for 36 hours. The product was filtered, washed with deionized (DI) water and dried at 250°F (120°C). The XRD pattern of the as-synthesized material showed the typical pure phase of ZSM-12 topology. The SEM, Figure 4 (b), of the as-synthesized material shows that the material was composed of much less densely packed agglomerates of small crystals with an average crystal size of < 0.05 microns. Argon-physisorption demonstrates that the H-form of the resulting product shows an of external surface area of about 20 % (as compared to total surface area) and an increased medium mesopore radius of about 39 Å (Figure 4 (a)) as compared to the product of Example 3.

## Claims

1. A process for synthesizing a porous, crystalline material having the framework structure of ZSM-12 of the formula:
(n)YO₂:X₂O₃
wherein X is a trivalent element, Y is a tetravalent element and n is between 80 and 250, the process comprising:
(a) preparing a mixture capable of forming said material, said mixture comprising sources of alkali or alkaline earth metal (M), an oxide of trivalent element (X), an oxide of tetravalent element (Y), hydroxyl (OH⁻) ions, water and tetraethylammonium cations (R), wherein said mixture has a composition, in terms of mole ratios, within the following ranges:
YO₂/X₂O₃ = 100 to 300;
H₂O/YO₂ = 5 to 15;
OH⁻/YO₂ = 0.10 to 0.30;
M/YO₂ = 0.1 to 0.2; and
R/YO₂ = 0.10 to 0.20;
(b) reacting said mixture at a temperature of at least 300°F (149°C) to form crystals of said material; and
(c) terminating (b) after a period of time of less than 50 hours and recovering said crystalline material.

2. The process of claim 1, wherein said temperature is 300°F (149°C) to 330°F (166°C) and preferably is 320°F (160°C).

3. The process of claim 1 or claim 2, wherein (b) is terminated after a period of time of less than or equal to 40 hours, preferably from 30 hours to 40 hours.

4. The process of any preceding claim, wherein said mole ratio of H₂O/YO₂ is between 10 and 14.

5. The process of any preceding claim, wherein said mole ratio of R/YO₂ is between 0.1 and 0.15.

6. The process of claim 1, wherein said mole ratio of M/YO₂ is greater than 0.1 and said temperature is less than 320°F (160°C).

7. The process of any preceding claim, wherein said X comprises aluminum and Y comprises silicon.

8. A process for the conversion of C₉+ aromatic hydrocarbons to xylenes comprising
(a) synthesizing a porous, crystalline material having the framework structure of ZSM-12 of the formula:
(n)YO₂: X₂O₃
wherein X is a trivalent element, Y is a tetravalent element and n is between 80 and 250, according to the process of anyone of claims 1 to 7, and
(b) contacting a feed comprising at least one C₉+ aromatic hydrocarbon under conversion conditions with a catalyst comprising the porous, crystalline material of step (a).

## Patentansprüche

1. Verfahren zur Synthese eines porösen, kristallinen Materials mit der ZSM-12-Gerüststruktur der Formel
(n)YO₂:X₂O₃
worin X ein dreiwertiges Element, Y ein vierwertiges Element und n zwischen 80 und 250 ist, wobei bei dem Verfahren
(a) ein Gemisch hergestellt wird, das imstande ist, das Material zu bilden, wobei das Gemisch Quellen eines Alkali- oder Erdalkalimetalls (M), ein Oxid eines dreiwertigen Elements (X), ein Oxid eines vierwertigen Elements (Y), Hydroxidionen (OH⁻), Wasser und Tetraethylammoniumkationen (R) umfasst, wobei das Gemisch eine Zusammensetzung in den folgenden Bereichen aufweist, angegeben in Molverhältnissen:
YO₂/X₂O₃ = 100 bis 300,
H₂O/YO₂ = 5 bis 15,
OH⁻/YO₂ = 0,10 bis 0,30,
M/YO₂ = 0,1 bis 0,2 und
R/YO₂ = 0,10 bis 0,20,
(b) das Gemisch bei einer Temperatur von mindestens 300°F (149°C) umgesetzt wird, um Kristalle des Materials zu bilden, und
(c) Schritt (b) nach einer Zeitspanne von weniger als 50 Stunden beendet wird und das kristalline Material gewonnen wird.

2. Verfahren nach Anspruch 1, wobei die Temperatur 300°F (149°C) bis 330°F (166°C) beträgt, bevorzugt 320°F (160°C).

3. Verfahren nach Anspruch 1 oder Anspruch 2, wobei Schritt (b) nach einer Zeitspanne von 40 Stunden oder weniger beendet wird, bevorzugt 30 Stunden bis 40 Stunden.

4. Verfahren nach einem der vorhergehenden Ansprüche, wobei das Molverhältnis von H₂O/YO₂ zwischen 10 und 14 beträgt.

5. Verfahren nach einem der vorhergehenden Ansprüche, wobei das Molverhältnis von R/YO₂ zwischen 0,1 und 0,15 beträgt.

6. Verfahren nach Anspruch 1, wobei das Molverhältnis von M/YO₂ größer als 0,1 ist und die Temperatur geringer als 320°F (160°C) ist.

7. Verfahren nach einem der vorhergehenden Ansprüche, wobei X Aluminium und Y Silizium umfasst.

8. Verfahren zur Umwandlung von aromatischen C₉+ Kohlenwasserstoffen in Xylole, bei dem
(a) ein poröses, kristallines Material mit der ZSM-12-Gerüststruktur der Formel
(n) YO₂:X₂O₃
gemäß dem Verfahren eines der Ansprüche 1 bis 7 synthetisiert wird, worin X ein dreiwertiges Element, Y ein vierwertiges Element und n zwischen 80 und 250 ist, und
(b) ein Einsatzmaterial, das mindestens einen aromatischen C₉+ Kohlenwasserstoff umfasst, unter Umwandlungsbedingungen mit einem Katalysator in Kontakt gebracht wird, der das poröse, kristalline Material aus Schritt (a) umfasst.

## Revendications

1. Procédé de synthèse d'une matière poreuse cristalline répondant à la structure de réseau de ZSM-12 de formule :
**(n)YO₂: X₂O₃**
où X représente un élément trivalent, Y représente un élément tétravalent et n est compris entre 80 et 250, le procédé comprenant :
(a) la préparation d'un mélange capable de former ladite matière, ledit mélange comprenant des sources d'un métal alcalin ou alcalino-terreux (M), un oxyde d'un élément trivalent (X), un oxyde d'un élément tétravalent (Y), des ions hydroxyle (OH⁻), de l'eau et des cations tétraéthylammonium (R), où ledit mélange présente une composition, en termes de rapports molaires, incluse dans les intervalles suivants :
YO₂/X₂O₃ = 100 à 300 ;
H₂O/YO₂ = 5 à 15 ;
OH⁻/YO₂ = 0,10 à 0,30 ;
M/YO₂ = 0,1 à 0,2 ; et
R/YO₂ = 0,10 à 0,20 ;
(b) la réaction dudit mélange à une température d'au moins 149 °C (300 °F) pour former des cristaux de ladite matière ; et
(c) l'arrêt de (b) après une durée de moins de 50 heures et la récupération de ladite matière cristalline.

2. Procédé selon la revendication 1, où ladite température est de 149 °C (300 °F) à 166 °C (330 °F) et préférentiellement de 160 °C (320 °F).

3. Procédé selon la revendication 1 ou la revendication 2, où (b) est arrêtée après une durée inférieure ou égale à 40 heures, préférentiellement comprise entre 30 heures et 40 heures.

4. Procédé selon l'une quelconque des revendications précédentes, où ledit rapport molaire de H₂O/YO₂ est compris entre 10 et 14.

5. Procédé selon l'une quelconque des revendications précédentes, où ledit rapport molaire de R/YO₂ est compris entre 0,1 et 0,15.

6. Procédé selon la revendication 1, où ledit rapport molaire de M/YO₂ est supérieur à 0,1 et ladite température est inférieure à 160 °C (320 °F).

7. Procédé selon l'une quelconque des revendications précédentes, où ledit X comprend de l'aluminium et Y comprend du silicium.

8. Procédé de conversion d'hydrocarbures aromatiques en C₉+ en xylènes comprenant
(a) la synthèse d'une matière poreuse cristalline répondant à la structure de réseau de ZSM-12 de formule :
**(n)YO₂: X₂O₃**
où X représente un élément trivalent, Y représente un élément tétravalent et n est compris entre 80 et 250, selon le procédé selon l'une quelconque des revendications 1 à 7, et
(b) la mise en contact d'un courant d'alimentation comprenant au moins un hydrocarbure aromatique en C₉+ dans des conditions de conversion avec un catalyseur comprenant la matière cristalline poreuse de l'étape (a) .
